(19) Europäisches Patentamt

European Patent Office

Office européen des brevets

(11) Veröffentlichungsnummer: **0 272 366**
**B1**

(12) **EUROPÄISCHE PATENTSCHRIFT**

(45) Veröffentlichungstag der Patentschrift:
**08.08.90**

(21) Anmeldenummer: **87106886.2**

(22) Anmeldetag: **12.05.87**

(51) Int. Cl.⁵: **D04H 1/44**, A61F 13/20

(54) **Medizinischer Saugkörper mit Röntgenkontrastmittel und Verfahren zu seiner Herstellung.**

(30) Priorität: **02.12.86 DE 3641151**

(43) Veröffentlichungstag der Anmeldung:
**29.06.88 Patentblatt 88/26**

(45) Bekanntmachung des Hinweises auf die Patenterteilung:
**08.08.90 Patentblatt 90/32**

(84) Benannte Vertragsstaaten:
**BE DE FR GB IT NL SE**

(56) Entgegenhaltungen:
**EP-A- 0 160 560**
**GB-A- 1 099 153**
**GB-A- 1 103 564**

(73) Patentinhaber: **Firma Carl Freudenberg, Höhnerweg 2-4,**
**D-6940 Weinheim/Bergstrasse(DE)**

(72) Erfinder: **Groitzsch, Dieter, Dr.,**
**Hermann-Löns-Strasse 6 A, D-6945 Hirschberg(DE)**
Erfinder: **Klein, Bernhard, Dr., Hofwiese 9,**
**D-6943 Birkenau-Löhrbach(DE)**
Erfinder: **Schaut, Gerhard, Dr., Brundhildstrasse 9,**
**D-6944 Hemsbach(DE)**

# Beschreibung

Die Erfindung betrifft einen medizinischen Saugkörper gemäss dem Oberbegriff des Anspruchs 1, der in der Chirurgie einsetzbar ist, sowie ein Verfahren zu dessen Herstellung.

Es muß sichergestellt sein, daß derartige Saugkörper oder Tupfer nach einem chirurgischen Eingriff vollzählig wieder aus dem Körper des Patienten entfernt werden. Daher ist es üblich, die Materialien mit sich von der Farbe des Blutes deutlich unterscheidenden Farbmarkierungen zu versehen, z.B. blau oder grün gefärbten Filamenten, wobei die Farben noch reflektierend, phosphoreszierend, fluoreszierend oder irisierend sein können.

Bei aller Umsicht kann der Fall jedoch nicht vollständig ausgeschlossen werden, daß Tupfermaterial versehentlich in der Operationsstelle zurückbleibt. Um z.B. bei ungewöhnlichen post-operativen Beschwerden des Patienten klären zu können, ob diese etwa von zurückgelassenem Saugkörper-Material herrühren, ist es notwendig, chirurgische Tupfer an bestimmten Stellen mit für Röntgenstrahlen undurchlässigen Materialien bzw. Einlagen zu versehen, welche auf dem Röntgenbild im Zweifelsfalle als Muster erkennbar sind und somit einen entsprechend dem Obigen positiven Befund ermöglichen. Als für Röntgenstrahlen undurchlässige Materialien werden bevorzugt Bariumsulfat-Partikeln verwendet.

Die EP-A 160 560 beschreibt einen bindemittelfreien, durch Wasserstrahlen hohen Druckes faserverschlungenen und perforierten medizinischen Saugkörper aus Vliesstoff, in dessen Innerem sich mindestens ein Röntgenstrahlen absorbierendes Element (Röntgenkontrastmedium) als integraler Bestandteil des Saugkörpers zwischen den Faserschichten befindet.

Dieses Element wird durch es umschlingende Fasern in seiner Lage gehalten. Vorzugsweise besteht das Röntgenkontrastmedium aus mindestens einem Garn, Faserband oder Endlos-Monofilament mit einer polymeren, thermoplastischen Matrix, welche Bariumsulfat in Mengen um 60 Gew.-% eingelagert enthält. Die Matrix ist teilweise mit den sie umgebenden Vliesstoff-Fasern thermisch verbunden, d.h. die Fasern sind in die nachträglich durch Heißverpressen weichgemachte Matrix eingebettet und fixieren so das Monofilament oder Garn in seiner Lage.

Die Vliesstoffe können aus natürlichen oder synthetischen Fasern oder Gemischen daraus bestehen, welche wirr abgelegt sind. Die Matrix des Röntgenkontrastmediums besteht bei Monofilamenten bevorzugt aus Polyvinylchlorid, hat einen Durchmesser von etwa 0,6 mm und ist zusätzlich zum Bariumsulfat mit Pigmenten versetzt, die in ihrer Farbe deutlich mit Blut kontrastieren. In jedem Fall muß sie jedoch thermoplastische Eigenschaften besitzen, damit die sie umgebenden Fasern nachträglich eingeschweißt werden können.

Die Anordnung mehrerer Röntgenkontrastfäden in Mustern oder Reihen und deren Anzahl hängt davon ab, in welcher Größe und welcher Faltung der Saugkörper verwendet werden soll. Es muß nur gewährleistet sein, daß jeder gebrauchsfertige Tupfer eine genügende Anzahl an Kontrastfäden enthält. Es wird auch vorgeschlagen, Fadenbruchstücke einzuarbeiten. Durch die musterartige oder unterbrochene Anordnung unterscheidet sich ein irrtümlich etwa in der Wunde zurückgelassener Tupfer von ebenfalls kontrastmittelhaltigem, jedoch notwendigerweise im Körper verbleibendem Nahtmaterial.

Aus der DE-OS 2 600 185 ist ein Verfahren bekanntgeworden, bei dem ein medizinischer Tupfer mit einer innig und unverlierbar mit den Fasern verbundenen Einlage dadurch hergestellt wird, daß ein geschmolzenes Filament aus röntgenstrahlen-undurchlässigem, thermoplastischem Material auf ein sich bewegendes, saugfähiges Gewebe extrudiert und dabei in noch klebefähigem Zustand gegen das Gewebe angepreßt wird. Es entsteht ein zusammengedrückter Strang, in welchen die ihn umgebenden Fasern eingebettet sind. Ein nachträgliches erneutes Aufschmelzen des thermoplastischen Materials ist nicht notwendig. Eine Anleitung zur Auswahl geeigneter thermoplastischer Polymerer wird nicht gegeben.

Die bekannten Saugkörper besitzen die folgenden produkt- und auch verfahrensbezogenen Nachteile:

a) Die Klarheit des Kontrastes auf der Röntgenaufnahme ist von der Auftragsmenge des Kontrastmediums, der Dichte (dem prozentualen Anteil) der röntgenkontrastierenden Partikeln und der Strahlendosis abhängig. Um die Strahlungsbelastung des menschlichen Körpers möglichst niedrig zu halten, ist man bestrebt, die Strahlendosis so niedrig wie möglich zu halten. Damit zusammen hängt der Wunsch nach möglichst hohen Konstrastmittelgehalten in der Polymermatrix, wie 60 Gew.-% und mehr.

In die Matrix eingearbeitete, anorganische Partikeln, meist Bariumsulfat, in den genannten Mengen bewirken eine drastische Verhärtung und Verminderung der Flexibilität in der thermoplastischen Matrix. Der Effekt der Verhärtung ist derart stark ausgeprägt, daß beispielsweise ein Kontrastfilament nach DE-OS 2 600 185 oder mit einer PVC-Matrix gemäß EP-A 160 560 nur bei außergewöhnlich hohen Gehalten an Weichmachern überhaupt zufriedenstellend extrudiert (ersponnen) werden kann. Daher scheiden für Röntgenkontrast-Monofilamente mit hohen Bariumsulfat-Füllmengen diejenigen Matrix-Materialien aus, die nicht mit Weichmachern verschneidbar oder die nur durch Copolymerisatbildung innerlich weich gemacht sind.

b) Aber auch die mit den für sie unverzichtbaren Weichmachern versetzbaren Thermoplasten weisen bei der Anwendung in medizinischen Saugkörpern Nachteile auf:

Weichmacher diffundieren oder migrieren bekanntermaßen im Vliesstoff und können daher beim Kontakt mit Wunden toxisch wirken.

c) Die obengenannten Anmeldungen schlagen vor, den im Interesse einer sicheren Detektierung relativ dicken (0,64 mm) Röntgenkontrastfaden über seine gesamte Länge während der Ablage oder nachträglich thermisch zu fixieren. Diese Maßnahme führt jedoch erfahrungsgemäß infolge der Dicke des Filaments zu einer erheblichen Verschlechterung oder sogar Behinderung der (mehrfachen) Faltbarkeit des Saugkörpers. Eine nur punktuelle Verbindung hingegen reicht zur sicheren Fixierung des Kontrastfadens nicht aus; die hohe Füllung der Matrix mit Kontrastmittel verschlechtert nämlich deren Klebefähigkeit im angeschmolzenen Zustand erheblich.

d) Gewebemull bzw. Mullersatzvliesstoffe werden normalerweise unsteril angeliefert und in krankenhauseigenen Dampfsterilisatoren nach dem pulsierenden Druck-/Vakuum-Prinzip bei 134°C im Autoklaven sterilisiert. Die Dampfsterilisierbarkeit ist z.B. bei zellwollhaltigen Geweben oder Vliesstoffen gegeben. Ist die Matrix des Röntgenkontrastmediums jedoch aus thermoplastischen und zusätzlich weichgemachten Polymeren aufgebaut, so besteht die Gefahr des Verklebens der aufeinandergeschichteten Faltkompressen nach der Dampfsterilisation.

Es ist die Aufgabe der vorliegenden Erfindung, die genannten Nachteile mit der Angabe eines verbesserten gattungsgemäßen, medizinischen Saugkörpers zu überwinden, wobei auch ein neuartiges Verfahren aufgezeigt werden soll, diesen herzustellen.

— Die Polymermatrix des Kontrastmediums soll also frei von Weichmachern und dennoch weich, flexibel und elastisch sein;
— sie soll, ohne zu erweichen, in der Hitze dampfsterilisierbar sein;
— sie soll dennoch auch bei hohen Kontrastmittelgehalten gut extrudierbar sein;
— die Fixierung des Röntgenkontrastmediums in noch klebefähigem Zustand direkt nach der Extrusion soll auf der Oberfläche des Vliesstoffmaterials des Saugkörpers autogen und unverrückbar möglich sein, wobei die mehrfache Faltbarkeit des Saugkörpers nicht beeinträchtigt werden darf.

Unter dem Begriff "Röntgenkontrastmedium" wird hier und in den folgenden Ausführungen jede Art von Einlagerung einer Röntgenstrahlen absorbierenden Substanz, gewöhnlich Bariumsulfat, in die Polymermatrix verstanden. Das System kann dabei eine beliebige geometrische Form besitzen. Ferner wird zweckmäßig eine größere Anzahl von Kontrastmedien im Vliesstoff oder auf diesem verteilt werden, um die zuverlässige Detektierbarkeit mit Röntgenstrahlen sicherzustellen.

Die Lösung der gestellten Aufgabe besteht in einem gattungsgemäßen medizinischen Saugkörper mit den kennzeichnenden Merkmalen des Anspruchs 1 sowie in einem Verfahren zu seiner Herstellung gemäß den Kennzeichen des ersten Verfahrensanspruchs. Die Unteransprüche betreffen bevorzugte Varianten des erfindungsgemäßen Produktes bzw. Verfahrens.

Die Matrix des Röntgenkontrastmediums ist erfindungsgemäß frei von externen Weichmachern, weich, elastisch, flexibel und nicht thermoplastisch.

Erfindungsgemäß liegt das Kontrastmedium, fest mit der Mehrzahl der Nachbarfasern verschweißt, auf der Vliesstoffoberfläche. Durch entsprechendes Falten ist es somit möglich, daß in jeder erdenklichen Lage des im Ernstfall zu findenden Saugkörpers in einer Operationswunde Teile des oder der Kontrastmedien sich auf der dem Röntgendetektor zugewandten Seite befinden und so besonders leicht aufgespürt werden können. Das Risiko des Ablösens von Partikeln beim Gebrauch ist wegen der intensiven Bindung an die Vliesstoffasern so gut wie ausgeschlossen.

Zweckmäßig, jedoch wegen des geringen Anteils in der hydrophilen Saugkörper-Basis nicht notwendigerweise, kann das Matrix-Material hydrophil sein. Zugunsten einer erhöhten mechanischen Festigkeit sind jedoch, ohne zu erwartende Nachteile, hydrophobe polymere Matrices ebenso verwendbar, zumal dann der erfindungsgemäße Saugkörper problemlos dampfsterilisierbar ist, ohne zu erweichen. Das erfindungsgemäße Röntgenkontrastmedium ist genügend weich, flexibel und elastisch, um die Faltbarkeit des Saugkörpers nicht zu behindern.

Das Matrix-Material ist ein vernetztes Acrylat-Polymer oder ein linearer oder vernetzter Polyharnstoff, ein Polyurethanpolyharnstoff oder eine Mischung aus beiden: Diese Substanzen sind aus Präpolymeren herstellbar, welche mit Wasser ausreagieren und so ein besonders einfaches Herstellungsverfahren ermöglichen, worauf später noch näher eingegangen wird. Weitere Vorteile liegen in der besonderen Weichheit und Flexibilität dieser Kunststoffklasse, ohne jedoch eine übermäßige Erweichung beim Erhitzen (Dampfsterilisation) zu zeigen.

Im Interesse einer guten Identifizierbarkeit auch in tiefer liegenden Gewebeschichten des menschlichen Körpers enthält die Polymermatrix zweckmäßig 40 bis 70 Gew.-% an Kontrastmittel, vorzugsweise Bariumsulfat-Partikeln.

Das Röntgenkontrastmedium kann jede beliebige, zweckmäßig Form besitzen; bevorzugt ist in vielen Fällen seine Ausbildung als Monofilament, mit kreisförmigem Querschnitt und zweckmäßig einem Durchmesser von 0,2 bis 0,5 mm bzw. einem Titer von 150 bis 700 tex, um zuverlässig mit Röntgenstrahlen detektierbar zu sein.

Zur Unterscheidung des Kontrastmittels aus einem etwa vergessenen Tupfer von einem ebenfalls Kontrastmittel enthaltenden Nahtmaterial, welches notwendigerweise in der Wunde verbleiben muß, ist es vorteilhaft, das Kontrastmedium in Form von auffälligen Mustern oder Reihen anzuordnen.

Das Kontrastmedium ist zweckmäßig mit blauen oder grünen Farbstoffen versetzt, die als Pigmente oder gelöst vorliegen und gegebenenfalls selbstleuchtend sein können. Dadurch ist der Saugkörper auch nach intensiver Tränkung mit Blut bereits während der Wundversorgung stets in auffälliger Weise sichtbar.

Der erfindungsgemäße Saugkörper kann in gelochter oder ungelochter Ausführung und/oder durch Hitze prägeverfestigt vorliegen und aus den bekannten, für diese Produkte für zweckmäßig befundenen Fasern bzw. Fasermischungen bestehen, wobei im Interesse der Saugfähigkeit mehrheitlich, d.h. zweckmäßig um 70 Gew.-%, vorhandenen cellulosischen Fasern der Vorzug zu geben ist, beispielsweise Zellwolle, Baumwolle, Zellstoff oder Abmischungen hiervon. Die restlichen Fasermaterialien sind dann z.B., gegebenenfalls in Abmischung, Polyacrylnitril-Hohlfasern, vollsynthetische Heterofil-Fasern, Polyamide, Polyester und Polyolefine. Es sind von externen Bindemitteln freie, dreilagig aufgebaute Vliesstoffe mit je einer sehr leichten Außenschicht aus dampfsterilisierbaren, thermoplastischen Synthesefasern und einer relativ schweren Saugkörper-Mittelschicht denkbar, die eine nur geringe Neigung zur Wundverklebung besitzen, da sie thermisch geglättete Oberflächen aufweisen.

Das Flächengewicht des ungefalteten Saugkörpers liegt in den üblichen Bereichen von 30 bis 100 g/m², wobei meist die leichteren Ausführungen angestrebt werden.

Die Einbindung des Röntgenkontrastmediums in die Oberflächenfasern ist in jeder Ausführungsform der vorliegenden Erfindung so gut, daß die Matrix in keinem Falle zusätzlich mit der Vliesstoffbasis punktverschweißt zu werden braucht.

Nach dem erfindungsgemäße Verfahren wählt man als Matrix, welche mit der ausreichenden Menge an Röntgenkontrastmittel, gewöhnlich Bariumsulfat, versetzt ist, nicht ein thermoplastisches Polymeres, sondern ein reaktives, niedrigschmelzendes Präpolymeres mit chemisch reaktiven Gruppen.

In einer bevorzugten Alternative werden Präpolymere eingesetzt, die zwei bis fünf endständige Isocyanatgruppen tragen, welche mit Wasser im Überschuß unter Harnstoffbildung abreagieren können. Das Molekulargewicht der eingesetzten Urethan-Präpolymere liegt vorzugsweise zwischen 168 und 20000.

Als besonders vorteilhaft wurden Molekulargewichte zwischen 1200 und 8000 gefunden: Niedrigere Werte ergeben relativ steife Produkte, Gewichtsbereiche über 8000 erschweren in der Regel eine gute Verarbeitung, weil die Viskosität des Präpolymeren zu hohe Werte erreichen kann.

Der oben angegebene Gehalt an zwei bis fünf freien Isocyanatgruppen sollte nicht überschritten werden, um ein zu starkes Aufschäumen durch $CO_2$-Abspaltung bei der Reaktion zu vermeiden. Dies ist insbesondere dann zu berücksichtigen, wenn das Gewicht des Saugkörpers relativ niedrig ist, z.B. 35 bis 40 g/m², Isocyanatkomponente: Grundsätzlich können alle di- und polyfunktionellen Isocyanate eingesetzt werden. Zweckmäßig für den medizinischen Bereich sind jedoch aliphatische Di- und Triisocyanate, wie beispielsweise Hexamethylendiisocyanat und ein trifunktionelles Isocyanat, basierend auf einer Biuretstruktur aus Hexamethylendiisocyanat mit einem Molekulargewicht von 478.

Polyolkomponente:

Wasser als Reaktionspartner ist besonders bevorzugt, weil es preisgünstig verfügbar, ausreichend reaktiv und vor allem harnstoffbildend ist. Die Polyharnstoffstruktur ist wegen ihrer thermischen Beständigkeit während der Dampfsterilisation besonders erwünscht.

Die erforderliche Weichheit der endständige Isocyanatgruppen tragenden Präpolymeren kann dabei durch weiteren Zusatz der aus der Polyurethanchemie bekannten Polyether- und/oder Polyesterpolyole begünstigt werden, wie z.B. Polypropylenoxiddiol (Molekulargewicht ca. 3600), Polytetrahydrofurandiol (Molekulargewicht ca. 200) oder Polyester aus Adipinsäure und Butandiol (Molekulargewicht ca. 2000). Diese Polyole werden in das molekulare Gerüst des Isocyanatgruppen tragenden Präpolymeren eingebaut. Externe Weichmacher, die irritierend oder toxisch wirken könnten, werden somit überflüssig.

Um die Reaktivität der Isocyanate gegebenenfalls an die Verfahrensbedingungen anzupassen, können die bekannten Katalysatoren für Polyurethan eingesetzt werden.

Das Ausreagieren der Präpolymeren erfolgt erfindungsgemäß erst nach der Extrusion, also nach der Ausbildung der geometrischen Form, und nach dem Anhaften an den und dem Inkorporieren der benachbarten Vliesfasern des Saugkörpers.

Wenn keine chemische Auspolymerisation erwünscht ist, ist – als zweite Alternative – oligomeren organischen Acrylat-Präpolymeren der Vorzug zu geben, die mit Teilchen- oder Wellenstrahlen, gegebenenfalls unter Zusatz von Initiatorsubstanzen, vernetzbar sind. Beispiele sind oligomere Systeme aus Epoxid/Acrylat, Polyester/Urethan/Acrylat, Polyether/Acrylat oder Polyester/Acrylat. Es ist auch hier darauf zu achten, daß die Vernetzungsprodukte per se bereits weiche, elastische und flexible Eigenschaften besitzen, eine Forderung, deren Erfüllung dem damit befaßten Polymerfachmann keine Schwierigkeiten bereiten wird. Bei der Vernetzung mit energiearmen Strahlen (z.B. UV-Strahlen) muß zudem gewährleistet sein, daß diese durch das Fasersubstrat bis zur Matrixsubstanz auch durchdringen, z.B. durch nicht zu große Materialdicken.

Die flüssig-viskose Konsistenz des Präpolymeren auch bei Kontrastmittel-Gehalten von 40 bis zu 70 Gew.-% und bereits bei Temperaturen deutlich unter 80°C ermöglicht die sofortige intensive Verbindung mit den benachbarten Fasern nach dem Auftreffen auf das Saugkörper-Substrat. Nach dem Auspolymerisieren reicht diese Bindung aus, um das Kontrastmedium dauerhaft zu fixieren. Eine weitere, z.B. punktförmige Nachverschweißung der Matrix ist nicht notwendig.

Es wäre denkbar, daß das Präpolymer zum Zwecke der Viskositätserniedrigung mit einem Lösungsmittel versetzt ist. Eine solche Möglichkeit ist jedoch aus wirtschaftlichen Gründen wenig vorteilhaft, weil das Lösungsmittel nach der Inkorporation des Röntgenkontrastmittels in den Vliesstoff wieder entfernt und regeneriert werden muß. Als vorteilhafter haben sich dagegen lösungsmittelfreie Systeme erwiesen.

Die erfindungsgemäßen, weichmacherfreien, viskos-fließfähigen Präpolymeren der Matrixsubstanz für das Kontrastmittel vereinen den Vorteil eines fließfähigen Stoffes, hohe Mengen an Kontrastmittel einlagern zu können, mit dem Vorteil von Zellwolle, auch bei Temperaturen um 134°C wasserdampfsterilisierbar zu sein.

Wie bereits an anderer Stelle erwähnt, kann auf die Auswahl hydrophiler oder hydrophilierter Präpolymerer zugunsten einer höheren mechanischen Festigkeit ohne weiteres verzichtet werden, da die Feuchtigkeitsaufnahmefähigkeit wegen des anteilmäßig sehr hohen Gewichtes des Basisvliesstoffes ohnehin gewährleistet ist.

Das Kontrastmedium kann als Präpolymer nach Erfordernis in jedem Stadium der Saugkörperherstellung aufgebracht werden. Es ist beispielsweise möglich, diesen Verfahrensschritt erst während der Faltung (Konfektionierung) des Vliesstoffes zu einem mehrlagigen, gebrauchsfertigen Saugkörper vorzunehmen und danach zu vernetzen.

Matrix-Präpolymere, die mit Wasser vernetzbar sind, bieten sich ganz besonders für solche modernen, bindemittelfreien Vliesverfestigungsverfahren an, die zur mechanischen Verschlingung der Fasern hochenergetische Wasserstrahlen benutzen. Dieselben Vorteile bestehen, wenn nur eine Perforierung ohne Faserverschlingung mit energiearmen Wasserstrahlen durchgeführt wird. In beiden Fällen kann hier nach dem Absaugen überschüssigen Wassers die Restfeuchte unmittelbar zur Nachpolymerisation der Kunststoffmatrix genutzt werden, was eine besonders wirtschaftliche Methode darstellt. Zudem ist das Abspaltungsprodukt $CO_2$ nicht toxisch.

Das Röntgenkontrastmedium wird vorzugsweise in Form eines Endlos-Monofilamentes extrudiert. Dessen Durchmesser liegt zweckmäßig zwischen 0,2 und 0,5 mm. Üblich ist die Extrusion durch kreisrund gelochte Düsenplatten mit einer oder mehreren Reihen von Bohrungen für den Durchtritt des gefüllten Präpolymeren. Die Anzahl und der Abstand der Bohrungen sollen so gewählt werden, daß nach dem Schneiden und Falten des fertigen Saugkörper-Vliesstoffes noch zumindest ein Kontrast-Monofilament pro Kompresse enthalten ist:

Gefaltete Kompressen sind in den Formaten 5*5, 7,5*7,5, 10*10 und 10*20 cm üblich. Vliesstoffkompressen sind so gefaltet, daß sie aus vier Lagen bestehen. Es ergibt sich also hier für das kleinste Faltkompressen-Format von 5*5 cm eine quadratische Gesamtfläche im aufgefalteten Zustand von

$$\sqrt{5 \times 5 \times 4} \quad \times \quad \sqrt{5 \times 5 \times 4} \quad = 10 \times 10 \text{ cm.}$$

Die Bohrungen in den Düsenplatten müssen dementsprechend einen Abstand von maximal 10 cm, am besten genau 10 cm, im rechten Winkel zur Maschinenrichtung (Vliesfertigungsrichtung) haben.

Mit dem beschriebenen Verfahren und seinen Varianten wird ein Saugkörper für den medizinischen bzw. chirurgischen Bereich erhalten, der leicht mehrfach faltbar ist, der auf seiner Oberfläche fest angebunden ein Röntgenkontrastmittel in genügender Menge enthält, um einen deutlichen Kontrast im Negativ hervorzurufen, und der ohne weiteres in der Hitze dampfsterilisierbar ist. Das Kontrastmedium ist unverrückbar an die Saugkörperfasern gebunden und dessen Matrix trotz absoluter Bindemittelfreiheit in flüssig-viskosem, klebefähigem Zustand bereits bei niedrigen Temperaturen extrudierbar. Somit wird die gestellte Aufgabe vollinhaltlich gelöst.

## Patentansprüche

1. Medizinischer Saugkörper aus einem durch Faserverschlingung oder -verschmelzung verfestigten Vliesstoff aus mehrheitlich cellulosischen Fasern mit mindestens einem damit befestigten Röntgenkontrastmedium aus einer mit für Röntgenstrahlen undurchlässigem Material gefüllten, polymeren Kunststoffmatrix, in welche ihr benachbarte Fasern des Vliesstoffes eingebettet sind und welche das absorbierende Material in Mengen von mindestens 40 Gew.-% enthält, dadurch gekennzeichnet, daß die Kunststoffmatrix frei von externen Weichmachern ist und aus linearem oder vernetztem, nicht thermoplastischem Polyharnstoff, Polyurethanpolyharnstoff oder Mischungen daraus oder aus vernetzten Acrylatpolymeren besteht und daß das Röntgenkontrastmedium auf der Saugkörper-Oberfläche innig an deren Fasern angebunden vorliegt.

2. Saugkörper nach Anspruch 1, dadurch gekennzeichnet, daß die Matrix des Röntgenkontrastmediums 40 bis 70 Gew.-% Bariumsulfat-Partikeln enthält.

3. Saugkörper nach Anspruch 1 bis 2, dadurch gekennzeichnet, daß das Röntgenkontrastmedium ein Monofilament mit kreisförmigem Querschnitt ist.

4. Saugkörper nach einem der vorhergehenden Ansprüche, dadurch gekennzeichnet, daß das Röntgenkontrastmedium in Mustern oder Reihen angeordnet ist.

5. Verfahren zur Herstellung eines medizinischen Saugkörpers nach Anspruch 1, wobei man auf die Oberfläche eines bewegten, mehrheitlich cellulosische Fasern enthaltenden Vlieses einen Matrix-Strang aus viskosfließfähigem, klebefähigem polymerem Material aufextrudiert, das mindestens 40 Gew.-% für Röntgenstrahlen undurchlässiges Material enthält, wobei die Mehrzahl der dem Strang unmittelbar benachbarten Fasern bei dessen Auftreffen in noch fließfähiges Polymermaterial eingebettet bzw. von diesem umflossen werden und zuletzt eine Verfestigung des Vlieses durch Faserverschlingung oder -verschmelzung erfolgt, dadurch gekennzeichnet, daß man, ohne externe Weichmacher anzuwenden, als Polymer-Matrix ein niedrigschmelzendes Präpolymeres mit chemisch reaktiven Gruppen einsetzt und dieses erst nach der Auftragung auf das Vlies durch Vernetzung und/oder Kettenverlängerung zu einem weichen, elastischen und flexiblen Kunststoff polymerisiert.

6. Verfahren nach Anspruch 5, dadurch gekennzeichnet, daß das Präpolymer ein oligomeres organisches, durch Teilchen- oder Wellenstrahlung vernetzbares Acrylat-System ist.

7. Verfahren nach Anspruch 5, dadurch gekennzeichnet, daß man als Präpolymer ein Urethan-Oligomer mit einem Molekulargewicht zwischen 168 und 20000 und mit 2 bis 5 freien Isocyanatgruppen pro Molekül verwendet und dieses mit Wasser im Überschuß unter Harnstoffbildung reagieren läßt.

8. Verfahren nach Anspruch 7, dadurch gekennzeichnet, daß man Oligomere mit Molekulargewichten zwischen 1200 und 8000 einsetzt.

9. Verfahren nach Anspruch 7 oder 8, dadurch gekennzeichnet, daß die Vliesverfestigung durch Faserverschlingung mit Wasserstrahlen erfolgt, wobei die nach dem Absaugen des Wassers verbleibende Restfeuchte unter Wärmezufuhr für die Reaktion mit dem Präpolymeren genutzt wird.

10. Verfahren nach Anspruch 7 oder 8, dadurch gekennzeichnet, daß man den Vliesstoff durch niederenergetische Wasserstrahlen unter Vermeidung von Faserverschlingungen perforiert, wobei die nach dem Absaugen des Wassers verbleibende Restfeuchte unter Wärmezufuhr für die Reaktion mit dem Präpolymeren genutzt wird, und das Produkt anschließend einem Vliesverfestigungsverfahren unterwirft.

11. Verfahren nach Anspruch 5 bis 10, dadurch gekennzeichnet, daß man das Röntgenkontrastmedium als Endlos-Monofilament extrudiert.

## Claims

1. A medicinal absorbent wad formed from a nonwoven from mainly cellulosic fibres consolidated by fibre entanglement or fusion together with at least one attached X-ray contrast medium formed from a polymeric plastics matrix which is filled with a material which does not transmit X-rays, in which adjacent fibres of the nonwoven are embedded and which contains the absorbent material in amounts of at least 40% by weight, characterized in that the plastics matrix is free from external plasticizers and is composed of linear or crosslinked, non-thermoplastic polyurea, polyurethanepolyurea or mixtures thereof or of crosslinked acrylate polymers and in that the X-ray contrast medium is present on the surface of the absorbent wad in intimate attachment to the fibres thereof.

2. An absorbent wad according to claim 1, characterized in that the matrix of the X-ray contrast medium contains from 40 to 70% by weight of barium sulphate particles.

3. An absorbent wad according to claim 1 or 2, characterized in that the X-ray contrast medium is a monofilament having a circular cross-section.

4. An absorbent wad according to any one of the preceding claims, characterized in that the X-ray contrast medium is arranged in patterns or rows.

5. A process for preparing a medicinal absorbent wad according to claim 1 by extruding onto the surface of a moving web containing mainly cellulosic fibres a matrix strand of polymeric material which is capable of viscous flow and of adhesion and which contains at least 40% by weight of a material which does not transmit X-rays, the majority of the fibres immediately adjacent to the strand becoming embedded in or enveloped by still fluent polymer material on impingement thereon, and finally consolidating the web by fibre entanglement or fusion, characterized in that the polymer matrix used, which is used without an external plasticizer, is a low-melting prepolymer having chemically reactive groups and this is polymerized to a soft, elastic and flexible plastic by crosslinking and/or chain advancement only after it has been applied to the web.

6. A process according to claim 5, characterized in that the prepolymer is an oligomeric organic acrylate system which is crosslinkable by particle or wave radiation.

7. A process according to claim 5, characterized in that the prepolymer used is a urethane oligomer having a molecular weight between 168 and 20,000 and from 2 to 5 free isocyanate groups per molecule and this is allowed to react with excess water to form a urea.

8. A process according to claim 7, characterized in that oligomers having molecular weights between 1200 and 8000 are used.

9. A process according to claim 7 or 8, characterized in that the web is consolidated by fibre entanglement with water jets, the residual moisture remaining after the water has been sucked off being utilized for the reaction with the prepolymer by supplying heat.

10. A process according to claim 7 or 8, characterized in that the nonwoven is perforated with low-energy water jets while avoiding fibre entanglements, the residual moisture remaining after the water has been sucked off being utilized for the reaction with the prepolymer by supplying heat, and the product is then subjected to a web consolidation process.

11. A process according to any of claims 5 to 10, characterized in that the X-ray contrast medium is extruded as a continuous monofilament.

## Revendications

1. Corps absorbant médical constitué d'une nappe de fibres consolidée par fusion ou entremêlement des fibres et formée en majeure partie de fibres cellulosiques, avec au moins un agent de contraste radiographique qui y est fixé et qui est constitué d'une matrice de matière plastique polymère chargée d'une matière imperméable aux rayons X dans laquelle sont insérées les fibres voisines de la nappe de fibres et qui contient le matériau absorbant en quantités d'au moins 40% en poids, caractérisé en ce que la matrice de matière plastique est exempte de plastifiants externes et est constituée de polycarbamides, de polyuréthanes/polycarbamides linéaires ou réticulés et non thermoplastiques ou de mélanges de ceux-ci, ou de polymères acryliques réticulés, et en ce que l'agent de contraste radiographique est intimement fixé aux fibres de la surface du corps absorbant.

2. Corps absorbant selon la revendication 1, caractérisé en ce que la matrice de l'agent de contraste radiographique contient de 40 à 70% en poids de particules de sulfate de baryum.

3. Corps absorbant selon les revendications 1 et 2, caractérisé en ce que l'agent de contraste radiographique est un monofilament de section circulaire.

4. Corps absorbant selon l'une des revendications précédentes, caractérisé en ce que l'agent de contraste radiographique est disposé suivant un dessin ou en rangées.

5. Procédé de fabrication d'un corps absorbant médical selon la revendication 1, dans lequel on extrude un filament de matrice de matériau polymère visqueux et liquide et collant à la surface d'une nappe de fibres en mouvement contenant en majeure partie des fibres cellulosiques, ce matériau contenant au moins 40% en poids de substances imperméables aux rayons X tandis que la majeure partie des fibres immédiatement voisines du fil sont insérées dans le matériau polymère encore fluide au moment où elles viennent en contact avec lui ou sont entourées par celui-ci, tandis qu'une consolidation de la nappe de fibres a finalement lieu par fusion ou entremêlement des fibres, caractérisé en ce que l'on utilise comme matrice de polymère un prépolymère à bas point de fusion et avec des groupes chimiques réactifs sans employer de plastifiant externe et que ce polymère est polymérisé en une matière plastique molle, élastique et flexible après l'application sur la nappe de fibres et par réticulation et/ou allongement des chaînes.

6. Procédé selon la revendication 5, caractérisé en ce que le prépolymère est un système acrylique organique oligomère qui peut être réticulé par des jets de particules ou d'ondes.

7. Procédé selon la revendication 5, caractérisé en ce que l'on utilise comme prépolymère un oligomère d'uréthane avec un poids moléculaire compris entre 168 et 20.000 et avec de 2 à 5 groupes isocyanates libres par molécule, et que l'on fait réagir celui-ci avec de l'eau en excès, avec formation d'urée.

8. Procédé selon la revendication 7, caractérisé en ce que l'on utilise des oligomères dont le poids moléculaire est compris entre 1200 et 8000.

9. Procédé selon la revendication 7 ou 8, caractérisé en ce que la consolidation de la nappe de fibres est réalisée par entremêlement des fibres avec des jets d'eau, tandis que l'humidité résiduelle après l'aspiration de l'eau est utilisée pour la réaction avec le prépolymère avec apport de chaleur.

10. Procédé selon la revendication 7 ou 8, caractérisé en ce que l'on perfore la nappe de fibres par des jets d'eau à faible énergie en évitant l'entremêlement des fibres, tandis que l'humidité résiduelle restant après l'aspiration de l'eau est utilisée pour la réaction avec le prépolymère avec apport de chaleur et que le produit est ensuite soumis à un procédé de consolidation de la nappe de fibres.

11. Procédé selon les revendications 5 à 10, caractérisé en ce que l'on extrude l'agent de contraste radiographique sous forme d'un monofilament sans fin.